# EUROPÄISCHE PATENTSCHRIFT

(11) **EP 1 940 383 B1**
(45) Veröffentlichungstag und Bekanntmachung des Hinweises auf die Patenterteilung: **08.06.2011**
(21) Anmeldenummer: 06804817.2
(22) Anmeldetag: 19.10.2006
(51) Int. Cl.: A61K 31/335, A61K 9/00, A61K 9/70, A61P 17/00

(54) **BEHANDLUNG UND PRÄVENTION VON GUTARTIGEN PIGMENTMALEN (NAEVI) MIT ARTEMISININ UND SEINEN DERIVATEN**
TREATMENT AND PREVENTION OF BENIGN PIGMENTED MOLES (NAEVI) USING ARTEMISININE AND THE DERIVATIVES THEREOF
TRAITEMENT ET PREVENTION DE MANIFESTATIONS PIGMENTAIRES (NAEVUS) BENIGNES GRACE A L'UTILISATION D'ARTEMISININE ET DE SES DERIVES

(30) Priorität: 20.10.2005 CH 16862005; 30.03.2006 CH 514062006
(43) Veröffentlichungstag der Anmeldung: 09.07.2008
(73) Patentinhaber: Epipharm GmbH, 4054 Basel (CH)
(72) Erfinder: SIFT CARTER, Rosemarie, 4054 Basel (CH)
(74) Vertreter: Braun, André jr.
(86) Internationale Anmeldenummer: PCT/CH2006/000583
(87) Internationale Veröffentlichungsnummer: WO 2007/045116

(56) Entgegenhaltungen:
- EP-A- 0 428 773
- WO-A-95/34262
- US-A- 6 127 405
- GALAL AHMED M ET AL: "Antifungal activity of artemisinin derivatives." JOURNAL OF NATURAL PRODUCTS. AUG 2005, Bd. 68, Nr. 8, August 2005 (2005-08), Seiten 1274-1276, XP002412656 ISSN: 0163-3864
- TROMBERG JENNIFER ET AL: "Congenital melanocytic nevi needing treatment." DERMATOLOGIC THERAPY. 2005 MAR-APR, Bd. 18, Nr. 2, März 2005 (2005-03), Seiten 136-150, XP002412657 ISSN: 1396-0296

## Beschreibung

Die Erfindung betrifft die Behandlung von gutartigen Pigmentmalen (Naevi) der Haut und der Schleimhäute, insbesondere von Näevuszellnaevi, Lentignosen und Pigmentmalen der Schleimhäute mit lokal applizierten, insbesondere topischen, Formulierungen, und des Weiteren hierzu geeignete topische Formulierungen.

Unter dem Begriff Naevuszellnävi versteht man verschiedene gutartige Hautveränderungen, Pigmentmale (Muttermal, Leberfleck), die sich feingeweblich aus sogenannten Naevuszellen zusammensetzen. Naevuszellen sind eine Fehlentwicklung der normalen Pigment bildenden Zellen, der Melanozyten. Melanozytäre Naevi kommen in unterschiedlicher Anzahl, Grösse und Farbintensität fast bei jedem Menschen vor. Naevi können im Hautniveau liegende oder über das Hautniveau erhabene Pigmentmale (kugelförmig, gestielt oder aufliegend), punktförmig oder grossflächig, warzig, höckrig oder glatt, sein, und die Farbgebung reicht von hautfarben über braun bis schwarz. Die Anzahl erworbener melanozytärer Naevi nimmt im Laufe des Lebens zu. Naevuszellnaevi mit auffälliger Struktur haben eine erhöhte Entartungsgefahr

Aus einem Naevuszellnaevus kann sich ein malignes Melanom, der schwarze Hautkrebs, entwickeln. In mehr als 60 Prozent der Fälle entsteht ein malignes Melanom aus einem Naevuszellnaevus. In den letzten Jahrzehnten wurde ein deutlicher Anstieg der Inzidenz beim Melanom registriert.

Laut heutigem Stand des Wissens gibt es keine vorbeugende Behandlung und keine Therapie, die einer Entartung von Naevuszellnaevi entgegenwirkt. Naevi mit einem erhöhten Risiko zu entarten werden vor allem operativ entfernt. Die Laserbehandlung spielt eher bei kosmetischen Aspekten eine Rolle. Beide Methoden sind invasiv, mit bestimmten Risiken behaftet (Narbenbildung, Hautverfärbung usw.) und mit hohen Kosten verbunden.

Der Entstehung eines erworbenen Naevus geht immer ein kleines, teilweise mikroskopisch kleines, rotes Pünktchen (eine Blutung oder ein Hämangiom) voraus. Aus dieser Naevus-Vorstufe entwickelt sich häufig ein grösseres rotes, etwas erhabenes Mal. Aus den Naevus-Vorstufen entstehen dann die braunen Naevuszellnaevi, von unterschiedlicher Grösse, Braunfärbung und Struktur.

Artemisinin ist ein Sesquiterpen-Lacton mit einer Peroxid-Gruppe, das bislang hauptsächlich als systemisch wirkendes Antimalariamittel untersucht und eingesetzt wurde. Artemisinin ist in Wasser sehr schlecht löslich; es sind aber wasserlösliche Derivate von Artemisinin entwickelt worden. In EP-A-0 428 773 wird die systemische oder topische Verwendung von Artemisinin und Derivaten davon zur Behandlung von Schuppenflechte, viralen Krankheiten (Warzen, Dellwarzen und Schafpocken), ultraviolettinduzierten Krankheiten (polymorphe Lichtdermatose, "collagen vascular disease", prämalignante Keratosen, Bowen-Syndrom, Lentigo maligna, Basalzellkrebs, Schuppenzellkrebs und malignes Melanom), bläschenbildenden Hautkrankheiten und Hämorrhoiden beschrieben.

Galal et al. beschreiben die antimykotische Wirkung verschiedener Artemisininderivate gegen Candida albicans und Cryptoccccus neoformans in vitro (J. Nat. Prod. 2005, 68, 1274-1276).

U.S. Patent 6,127,405 beschreibt die Verwendung von α-Arteether als Wachstumsinhibitior für durch eine Mutation eines Gyrase-Gensgegen Chinolone resistente Bakterienstämme sowie als Antimykotikum.

WO 95/34262 A1 beschreibt die transdermale Anwendung von Artemisininderivaten in Form von Pflastern bei Behandlung oder Prophylaxe von Malaria.

Aufgabe der einfügen Erfindung ist die Bereitstellung von lokal applizierbaren, bevorzugt topischen, Formulierungen, die gegen gutartige Pigmentmale, insbesondere gegen melanozytäre Naevi, wirksam sind und so auch der Prävention von Hautkrebs dienen können.

Die Aufgabe wird erfindungsgemäss gelöst, indem eine Verbindung der Formel 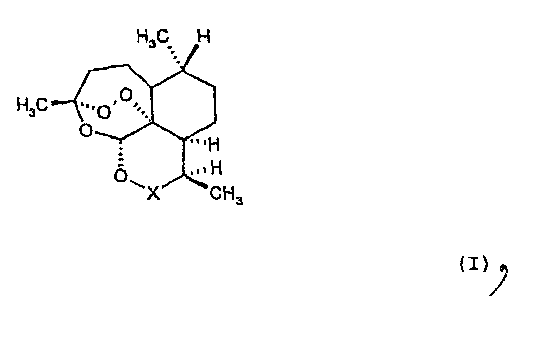 worin eine der in Anspruch 1 definierten Bedentungen hat, zur Herstellung einer lokal applizierbaren, insbesondere topischen, Formulierung gegen gutartige Pigmentmale verwendet wird.

Es wurde überraschenderweise gefunden, dass sich Pigmentmale der Haut, insbesondere diejenigen melanozytären Ursprungs, sehr früh erfolgreich mit oben genannten Wirkstoffen lokal, insbesondere mit topischen (z.B. kutanen) Zubereitungen, behandeln lassen und dass durch die Behandlung von Naevuszellnaevi mit einer Verbindung der Formel I die Prävention von Hautkrebs (insbesondere Basaliom oder Melanom) möglich wird. Es wurde des Weiteren gefunden, dass diese Wirkstoffe bei lokaler Anwendung auch in der Prävention von gutartigen Pigmentmalen, insbesondere von erworbenen Naevuszellnaevi, wirksam sind.

Im Rahmen der Erfindung werden als "gutartige Pigmentmale" insbesondere verstanden:
- Naevi, insbesondere der Naevuszellnaevus (banal oder dysplastisch; der Naevuszellnaevus wird oft auch melanozytärer Naevus genannt), darunter seine drei nach Entstehungsort unterscheidbaren Subtypen Junktions-Naevuszellnaevus (Grenzzone Oberhaut/Lederhaut), Compound-Naevuszellnaevus (Bindegewebe der Lederhaut) und dermaler Naevuszellnaevus (tiefe Schichten der Lederhaut), sowie seine nach Enstehungszeitpunkt unterscheidbaren Subtypen kongenitaler Naevuszellnaevus (= Muttermal) und erworbener Naevuszellnaevus. Eine Untergruppe von erworbenen Naevuszellnaevi sind Rezidivnaevi, die nach der chirurgischen Entfernung eines anderen gutartigen Muttermals entstehen.
- Lentiginosen (etwa Leberflecken = Lentigo simplex, Sonnenflecken = Lentigo solaris, Altersflecken = Lentigo senilis, PUVA-Lentigines) ;
- Störungen der Melaninpigmentierungen (etwa Sommersprossen = Ephelides); und
- Pigmentmale der Schleimhäute (etwa Bindehautnaevus am Auge, Naevus an Lippen, Mundschleimhaut oder Geschlechtsorganen).

Die Verbindungen I sind wirksam in der Behandlung aller oben erwähnten gutartigen Pigmentmale, insbesondere der erworbenenen oder kongenitalen Naevuszellnaevi. Unter den oben erwähnten Naevi haben die dysplastischen (atypischen) Naevuszellnaevi eine erhöhte Wahrscheinlichkeit, zu Hautkrebs zu entarten, und sind daher Naevi, die bevorzugt mit Verbindungen I zur Prävention von Hautkrebs behandelt werden

Für (C₁-C₆)Alkyl (per se oder in (C₁-C₆) Alkoxy) sind Methyl, Ethyl, n-Propyl, i-Propyl, n-Butyl, sec-Butyl, t-Butyl, n-Pentyl, sec-Pentyl, neo-Pentyl, n-Hexyl, sec-Hexyl und neo-Hexyl bevorzugt. Eher bevorzugt ist geradkettiges (C₁-C₃)Alkyl, besonders bevorzugt ist Methyl oder Ethyl; bevorzugt ist (C₁-C₃)Alkoxy, eher bevorzugt ist Methoxy, Ethoxy oder n-Propoxy. Für (C₂-C₆)Alkenyl ist (C₂-C₄)Alkenl, etwa Vinyl, Allyl, 1-Methylvinyl, 2-Methylvinyl, But-1-en-1-yl, But-2-en-1-yl, But-3-en-1-yl, But-1-en-2-yl, But-3-en-2-yl, 2,2-Dimethylvinyl und 1,2-Dimethylvinyl, bevorzugt. Für (C₂-C₆)Alkinyl sind etwa Ethinyl, Propargyl, Prop-1-in-1-yl, But-1-in-1-yl, But-2-in-1-yl, But-3-in-1-yl, But-3-in-2-yl, 3-Methylbut-1-in-1-yl, 3,3-Dimethylbut-1-in-yl, 1,1-Dimethylbut-2-in-1-yl und 1,1-Dimethylprop-2-in-1-yl bevorzugt. Für (C₃-C₈) Cycloalkyl siwd Cyclopropyl, Cyclobutyl, Cyclopentyl und Cyclohexyl bevorzugt. Für (C₆-C₂₄) Aryl ist (C₆-C₁₀)Aryl, etwa Phenyl, Naphth-1-yl und Naphth-2-yl, bevorzugt. Für (C₇-C₂₄) Aralkyl ist (C₇-C₁₂)Aralkyl, etwa Benzyl, Phenethyl, Naphth-1-ylmethyl und Naphth-2-ylmethyl, bevorzugt. Für (C₆-C₁₀) Aryloxy bevorzugt ist Phenoxy oder α- oder β-Naphthoxy.

"Artemisinyl" bezeichnet im Rahmen der Erfindung eine Gruppe der Formel I mit X = CH-die über die freie Valenz des Kohlenstoffs an den Sauerstoff oder den Stickstoff gebunden werden kann. "10-α-Dihydroartemisinyl" bedeutet im Rahmen der Erfindung -O-Artemisinyl, worin Artemisinyl die vorstehende Bedeutung hat.

Für Z sind Wasserstoff; geradkettiges oder verzweigtes (C₁-C₆) Alkyl; m- oder p-CH₂ (C₆H₄) COOM; COR³; SOR⁴; SO₂OM; SO₂NR⁷R⁸; SO₂NH-Artemisinyl und POR⁴R⁵ bevorzugt.

Für das pharmazeutisch annehmbare Kation M können beispielhaft Kationen von Alkalimetallen, z.B. Lithium, Natrium oder Kalium, oder Erdalkalimetallen, z.B. Magnesium oder Calcium, Ammonium und H⁺N (R^{x}R^{y}R^{z}) genannt werden, wobei R^{x}, R^{y}, R^{z} unabhängig voneinander Methyl oder Ethyl sein können.

für Z als COR³ ist R³ bevorzugt ein geradkettiger oder verzweigter (C₁-C₆) Alkyl- oder (C₁-C₆)Alkoxy-Rest; oder-(CH₂)ₙ-COOM, wobei M bevorzugt Wasserstoff, Natrium, Kalium oder Ammonium ist.

Für Z als SO₂OM ist M bevorzugt Alkalimetall, Erdalkalimetall oder Ammonium.

Für Z als POR⁴R⁵ ist bevorzugt, dass R⁴ und R⁵ aus OM und geradkettigem oder verzweigtem (C₁-C₆)Alkoxy ausgewählt sind. Eher bevorzugt ist eines von R⁴ und R⁵ OM, wobei M insbesondere Natrium, Kalium oder Ammonium ist, und das andere geradkettiges oder verzweigtes (C₁-C₆)Alkoxy oder OH.

Die Verbindungen I sind bekannt (Z.B. Artemisinin, dihydroartemisinin, Artesunat, Artemether, Arteethus, oder Artelinsäure) oder in Analogie zu bekannten Verbindungen I herstellbar. Die Verbindungen, bei denen X = CHOZ ist, mit Z verschieden von Wasserstoff, oder bei denen X = CHNRZ ist, werden im Folgenden als "Derivate des Dihydroartemisinins" bezeichnet. (z.B. Artemisinin, Dihydroartemisinin, Artesunat, Arternether, Arteether oder Artelinsäure)

- Die Derivate des Dihydroartemisinins, bei denen X = CHOZ bzw. CHNRZ ist, R die bei Formel (I) angegebene Bedeutung hat, Z = COR³ oder CSR³ bedeutet und R³ geradkettiges oder verzweigtes (C₁-C₆)Alkoxy oder (C₆-C₁₀)Aryloxy ist, können durch Umsetzen von Dihydroartemisinin bzw. dem Derivat des Dihydroartemisinins mit X = CHNRH mit dem geeigneten Chlorkohlensäure- (C₁-C₆) alkylester oder -(C₆-C₁₀)arylester oder Chlorthiokohlensäure-(C₁-C₆)alkylester oder -(C₆-C₁₀)arylester und einer Base hergestellt werden.

Bei den Verbindungen mit X = CHOZ oder CHNRZ kann die Konfiguration an dem C₁₀-Atom des Sesquiterpengerüstes (R) oder (S) sein. Die Verbindung I kann auch in Form einer C₁₀-Epimerenmischung eingesetzt werden.

Bevorzugt sind als Wirkstoffe der Formel I Artemisinin, Dihydroartemisinin, die carboxylgruppenhaltigen Derivate (insbesondere Artesunat), Artemether, Arteether, das Propylcarbonat des Dihydroartemisinins und Artelinsäure. Besonders bevorzugt sind Artemisinin, Dihydroartemisinin und Artesunat.

Die Verbindungen I können einzeln oder als Kombination von zwei oder mehreren dieser Verbindungen eingesetzt werden.

Die Verbindungen I, insbesondere Artesunat, sind auch in der Prävention von erworbenen Naevuszellnaevi wirksam. Zur Prävention von Naevuszellnaevi werden die Verbindungen I, insbesondere Artesunat, grossflächig auf die gesamte Haut, auf Hautzonen mit erhöhter Wahrscheinlichkeit für die Bildung von Naevi, oder auf bereits sichtbare Naevus-Vorstufen aufgetragen. Hautzonen mit erhöhter Wahrscheinlichkeit für die Bildung von Naevi sind einerseits Hautpartien, die häufiger ultravioletter Strahlung ausgesetzt waren. Anderserseits sind solche Hautzonen oft in der Umgebung eines bereits ausgebildeten Naevuszellnaevus (z.B. in einem Radius von typisch bis zu 5 cm um den bereits vorhandenen Naevus) vorhanden. Die Verbindungen I, insbesondere Artesunat, haben dabei die weitere vorteilhafte Eigenschaft, dass sie bereits vorhandene Naevus-Vorstufen, die noch so schwach sind, dass man sie von blossem Auge kaum erkennt, sichtbar machen. Die noch unsichtbaren Naevus-Vorstufen bilden unter Einwirkung der Verbindungen I zunächst kleine rote Pünktchen, die nach einigen Tagen dunkel bis schwarz werden und teilweise wie dunkle, in Poren sitzende Kristalle aussehen. In günstigen Fällen kann sich also eine auflichtmikroskopische Voruntersuchung der fraglichen Hautstelle erübrigen.

Zur Anwendung können die Wirkstoffe in eine für lokale, insbesondere topische (kutane), Anwendung geeignete Formulierung formuliert werden. Die Konzentration der Wirkstoffe in den Formulierungen ist nicht besonders kritisch. Es können Formulierungen mit einer Konzentration von etwa 0,1 bis etwa 40 Gewichtsprozent hergestellt werden. Für die Behandlung von Naevuszellnaevi (kongenital oder erworben) enthalten die Formulierungen bevorzugt etwa 5 bis etwa 20, besonders bevorzugt etwa 10, Gewichtsprozent, Wirkstoff. Für die Prävention von erworbenen Naevuszellnaevi enthalten die Formulierungen bevorzugt bis zu etwa 5, eher bevorzugt etwa 1 bis etwa 5, Gewichtsprozent der Verbindung. Die genaue therapeutisch benötigte Wirkstoffmenge ist von der Wirksubstanz selber, der verwendeten Grundlage, der galenischen Form (etwa Salbe, Suspension, Paste, Pflaster, Creme, Gel, Lösung) sowie von verwendeten Zusatzstoffen abhängig und kann vom Fachmann durch einfache Wirksamkeitstests festgestellt werden.

Die Behandlungsdauer von bestehenden Naevuszellnaevi (kongenital oder erworben) hängt von Art, Grösse, Struktur, Pigmentierung und Alter des Nävus ab. Vorzugsweise werden die Behandlungen bei hohen Konzentrationen an Verbindung I zyklisch durchgeführt. Erste Reaktionen werden häufig nach den ersten Behandlungstagen sichtbar. Bis zu einer eindeutigen Besserung oder Veränderung, was sich in einer Verblassung oder einem Verschwinden des Naevuszellnaevus äussert, kann es zwei bis mehrere Monate dauern. Dieser Zeitraum kann bei älteren Patienten länger sein, da die Erneuerung der Epidermis mit zunehmendem Alter bedeutend länger dauert.

Für die Prävention von erworbenen Naevuszellnaevi ist eine 2-3malige Anwendung ausreichend. Grössere Naevus-Vorstufen werden bevorzugt länger bis zum Verblassen oder Verschwinden behandelt.

Der Wirkstoff sollte je nach Therapieansatz unterschiedlich tief in die Haut eindringen: - Für die Behandlung von Naevuszellnaevi (kongenital oder erworben) dringt der Wirkstoff, je nach Typ und Alter des Naevus, bevorzugt bis in die obere Lederhaut ein. - Für die Prävention von erworbenen Naevuszellnaevi dringt der Wirkstoff bevorzugt durch die Oberhaut bis zur Junktionszone, der Grenze zwischen Ober- und Lederhaut, ein.

Als Formulierungsgrundlage für die Wirkstoffe der Formel I eignen sich sämtliche diesen Wirkstoffen gegenüber inerte, für lokale Formulierungen übliche, Grundlagen. Insbesondere können solche Grundlagen für topische Formulierungen Vaseline, Fette, Wachse, Fettsäureester, Paraffine, Öle, Silikone und seine polymeren sein. Bevorzugt werden die Wirkstoffe mit etwa 60 bis etwa 99,9, eher bevorzugt mit etwa 80 bis etwa 95, Gewichtsprozent an Formulierungsgrundlage formuliert. Werden hydrophile / wässrige topische Formulierungsgrundlagen verwendet, wie Hydrogele oder Cremen, können die Wirkstoffe durch Nanoverkalpselung, Einschluss in Liposomen oder Komplexbildung mit zum Beispiel Cyclodextrinen vor Zersetzung geschützt werden.

Topische Formulierungen mit einer wasserfreien, einphasigen Grundlage, z.B. einer reinen Fettphase, werden nach dem Deutschen Arzneibuch als Salben bezeichnet. Salben, in denen die Wirkstoffe erfindungsgemäss verwendet werden, bestehen somit aus einer solchen Salbengrundlage, die den oder die fein verteilten Wirkstoffe zur Anwendung an der Haut enthält.

Die Formulierungsgrundlage kann, wenn die topische Formulierung eine Salbe sein soll, bevorzugt aus lipophilen Bestandteilen mit einem N-Octanol/Wasser-Partitionskoeffizienten bei Raumtemperatur von etwa 1 bis etwa 10⁵, eher bevorzugt von etwa 10 bis etwa 10⁵, besonders bevorzugt von etwa 50 bis etwa 10⁴, bestehen. Beispiele für die Formulierungsgrundlage sind hier vaseline, Fette, Wachse, Fettsäureester, Paraffine, Öle, Silikone und seine Polymeren (z.B. Polydialkylsiloxane, Silikon-Elastomere, Silikon-Wachse, Silikon-Emulsifier).

Im Gegensatz zur Salbe werden Zweiphasensysteme (Wasser- und Fettphase) als Creme benannt. Die Verbindungen I lassen sich auch als Creme formulieren. Für die Fettphase kommen dieselben Stoffe in Betracht, wie sie für die Salbengrundlage exemplifiziert wurden. Die Wasserphase kann wahlweise Puffersubstanzen enthalten, die einen hautverträglichen pH der Wasserphase bewirken, oder sie kann auch bekannte gelbildende Polymere, wie Hydroxypropylmethylcellulose, Carboxymethylcellulose, Polyvinylalkohol mit Quervernetzern (etwa Borax oder mehrwertige Metallkationen, wie Mg²⁺ oder Ca²⁺) u.ä., enthalten. Zur Emulgierung können übliche hautverträgliche oberflächenaktive Stoffe, wie Fettsäuremono- und -diglyceride, PEG-40-hydriertes Rizinusöl (Cremophor ®) oder Lecithin, verwendet werden.

Als Hilfsstoffe für topische Formulierungen sind übliche Penetrationsbeschleuniger (wie Dimethylacetamid, Dimethylformamid, Propylenglykol, Fettalkohole, Triethanolamin, Dimethylsulfoxid, Azone u.a.), Keratolytika zur Verbesserung der Wirksamkeit (wie Salicylsäure, Harnstoffe, Retinoide) und Konservierungsmittel möglich. Zusatzstoffe dienen im Allgemeinen zur Verbesserung der Wirksamkeit, Stabilität, Haltbarkeit und Konsistenz einer galenischen Darreichungsform.

Die Verbindungen I werden in topischen Formulierungen bevorzugt im Wesentlichen frei von penetrationsverstärkenden Stoffen formuliert. Die Verbindungen I werden auch bevorzugt im Wesentlichen frei von (C₅-C₁₉)Monocarbonsäuren, ihren Estern und ihren Amiden formuliert. Unter "im Wesentlichen frei" wird im Rahmen der Erfinderung verstanden, dass die topische Formulierung weniger als , bevorzugt weniger als 0,1, Gewichtsprozent an penetrationsverstärkenden Stoffen aufweist.

Als Verbindungen I für Pasten, Salben, Cremen, Lösungen, Gele, Sprays oder Suspensionen sind alle Derivate mit einer Carboxylgruppe, insbesondere Artesunat, bevorzugt. Die Carboxylgruppe kann dabei wahlweise in der Form des Alkalimetall-, Erdalkalimetall- oder Ammoniumsalzes vorliegen.

Die Wirkstoffe werden zur Anwendung auf oder in die Haut bevorzugt in Form einer topischen Formulierung, insbesondere als Paste, Salbe, Suspension, Lösung, Gel, Spray oder Creme, besonders bevorzugt als Salbe, auf ein Pflaster aufgetragen. Dieses Pflaster kann wahlweise ein die topische Formulierung aufnehmendes oder aufsaugendes Material aufweisen. Der Wirkstoff kann auch direkt in einem inerten Kleber des Pflasters suspendiert oder gelöst werden. Auf diese Weise können Wirkstoffe direkt und über längere Zeit mit der zu behandelnden Stelle in Kontakt treten. Zusätzlich kommt es zu einem Okklusionseffekt, was die Wirkstoffpenetration verbessert.

Weitere Darreichungsformen der Wirkstoffe sind Pasten, Lösungen, Suspensionen, Gele und Sprays. Die halbfesten oder flüssigen Formulierungen der Wirkstoffe können auch in Form eines stiftfs (z.B. filzstiftähnlich zur genauen Dosierung) oder Rollers (mit Wirkstoff in geeigneter Grundlage, Lösung, Suspension, Salbe, Creme) vorliegen.

Weitere Beispiele von lokalen, erfindungsgemäss einsetzbaren Applikationsformen für die Verbindungen I sind Applikatoren, die das Eindringen der Verbindungen in die Haut oder Schleimhaut mittels Ultraschall, elektrischer Felder oder Mikronadeln bewirken. Applikatoren, die Ultraschall verwenden und erfindungsgemäss einsetzbar sind, sind etwa in US-B-6,908,448 offenbart. Applikatoren, die zur Applikation der Wirkstoffe elektrische Felder anwenden (die mithin das Prinzip der Iontophorese einsetzen), sind seit langem vorbekannt. Sie sind erfindungsgemäss geeignet für diejenigen Wirkstoffe der Formel (I), die salzartig sind, also etwa solche, bei denen X CHOZ oder CHNRZ ist, wobei Z aus m- und p-CH₂(C₆H₄)COOM, SO₂OM und POR⁴R⁵ ausgewählt ist, worin eines von R⁴ und R⁵ OM und das andere geradkettiges oder verzweigtes (C₁-C₆)Alkoxy oder OH ist, und M ein pharmazeutisch annehmbares Kation bedeutet. Für vorbekannte Applikatoren mit Mikronadeln zur erfindungsgemässen lokalen Applikation von Wirkstoffen auf die Haut kann auf US-A-2005/065463 verwiesen werden.

Ein weiteres Beispiel für eine erfindungsgemäss einsetzbare lokale Applikationsform ist eine Technik, bei der die Haut an einer zu behandelnden Stelle mittels eines Saugnapfs angehoben wird und auf der erhöhten Stelle der Haut ein Teil der Schichtdicke der Lederhaut mechanisch, etwa mit einer Klinge, abgetragen wird. Diese Stelle der Haut mit teilweise abgetragener Lederhaut ist durchlässiger für die Verbindungen I und erlaubt an dieser Stelle die lokale Behandlung tiefer liegender Schichten der Lederhaut. Die hierfür erforderlichen Geräte sind in WO-A-95/15783 beschrieben.

Eine risikoarme, nicht invasive vorbeugende bzw. therapeutische Behandlung von erworbenen Naevuszellnaevi bzw. von erworbenenen oder kongenitalen Naevuszellnaevi mit Artemisinin und seinen Derivaten (Dihydroartemisinin, Arteether, Artemether, Artesunat, semisynthetischen Derivaten und synthetisch analogen Verbindungen) stellt einen gewaltigen Fortschritt in der Behandlung von Naevuszellnaevi dar und könnte das Hautkrebsrisiko drastisch verringern. Die Erfindung ist somit nicht nur medizinisch, sondern auch sozioökonomisch von grosser Bedeutung. Bei den beschriebenen topischen Behandlungen mit den Verbindungen I, insbesondere Artesunat, wurden keine allergischen Hautreaktionen auf die Verbindungen beobachtet. Bemerkenswert ist auch, dass gesundes Gewebe nicht geschädigt wird und die Behandlung schmerzfrei und einfach ist.

Angesichts der bis jetzt erzielten Resultate; kann davon ausgegangen werden, dass die lokale, insbesondere topische, Therapie mit Artemesinin und seinen Derivaten sehr effektiv ist und als Prävention und Behandlung von Naevi über längere Zeit gesehen kostengünstiger und risikoärmer als traditionelle und invasive Behandlungsmethoden ist.

Die Erfindung wird durch die Beispiele weiter veranschaulicht.

### Beispiel 1: Topische Formulierung

Es wurden 3 g Artesunat mit 27 g Excipial® Fettsalbe homogen verrührt.

### Beispiele 2a-8f: Topische Formulierungen

Es wurden unterschiedliche Mengen Artesunat (s. Tabelle 1) in die verschiedenen Grundlagen eingearbeitet. Teilweise wurden den Formulierungen auch oberflächenaktive Stoffen zugegeben.

**Tabelle 1:**

| Bsp. Nr. | Artesunat (in g) | Additive | Grundlage q.s. ad 100 g |
|---|---|---|---|
| 2a-2h | 2a: 0,1 | - | weisse Vaseline |
| | 2b: 0,5 | | |
| | 2c: 1,0 | | |
| | 2d: 5,0 | | |
| | 2e: 10,0 | | |
| | 2f: 15,0 | | |
| | 2g: 30 | | |
| | 2h: 40 | | |
| 3a-3h | 3a: 0,1 | Polysorbat 0,5 g; | weisse Vaseline |
| | 3b: 0,5 | Macrogol 2000- | |
| | 3c: 1,0 | Stearat 0,5 g | |
| | 3d: 5,0 | PEG-40-sorbitanpero- | |
| | 3e: 10,0 | leat 0,5 g | |
| | 3f: 15,0 | | |
| | 3g: 30 | | |
| | 3h: 40 | | |
| 4a-4h | 4a: 0,1 | - | Bienenwachs |
| | 4b: 0,5 | | |
| | 4c: 1,0 | | |
| | 4d: 5,0 | | |
| | 4e: 10,0 | | |
| | 4f: 15,0 | | |
| | 4g: 30 | | |
| | 4h: 40 | | |
| 5a-5h | 5a: 0,1 | Soja-Lecithin 2 g | Paraffin |
| | 5b: 0,5 | | |
| | 5c: 1, 0 | | |
| | 5d: 5,0 | | |
| | 5e: 10,0 | | |
| | 5f: 15,0 | | |
| | 5g: 30 | | |
| | 5h: 40 | | |
| 6a-6h | 6a: 0,1 | Macrogol 2000- | Rapsöl |
| | 6b: 0,5 | Stearat 2 g | |
| | 6c: 1,0 | | |
| | 6d: 5,0 | | |
| | 6e: 10,0 | | |
| | 6f : 15,0 | | |
| | 6g: 30 | | |
| | 6h: 40 | | |
| 7a-7f | 7a: 0,1 | Isopropylmyristat 1 g | Dekamethylcyclopentasiloxan (19 g) |
| | 7b: 0,5 | | |
| | 7c: 1,0 | | Silikon-Elastomer-Gel (ad 100 g) |
| | 7d: 5,0 | | |
| | 7e: 10,0 | | |
| | 7f: 15,0 | | |
| 8a-8f | 8a: 0,1 | - | Dekamethylcyclopentasiloxan 25 g |
| | 8b: 0,5 | | |
| | 8c: 1,0 | | Mineralöl ad 100 g |
| | 8d: 5,0 | | |
| | 8e: 10,0 | | |
| | 8f: 15,0 | | |

### Beispiele 9a-9i: Topische (kutane) Anwendungen in der Behandlung oder Prävention von Pigmentmalen

a) Ein 13 jähriger Junge mit einem erhabenen, dunkelbraunen Muttermal (dysplastischer Naevuszellnaevus auf der Brust) mit unebener Struktur von ca. 1,2 cm Durchmesser wurde 2-3 Mal wöchentlich mit der 10%-igen salbe von Bsp. 2e behandelt. Das Muttermal war nach 2 Wochen hellbraun mit ein paar dunklen, kleinen punktförmigen Stellen, die war "auskristallisierter" Farbstoff aussahen. Das Muttermal war trocken und schuppig und sah aus, als würde es sich von "innen" her zurückbilden.
b) Eine Frau mit 3 schwarzen, über die Hautoberfläche erhabenen Muttermalen (Junktionsnaevi am Rumpf) mit einem Durchmesser von 0,5 bis 1,0 cm wendete die 10%ige salbe von Bsp. 2e 3mal wöchentlich unter Okklusion (als Pflaster) über Nacht an. Nach einer Woche war in jedem Muttermal eine dunkle Stelle zu erkennen. Nach 3 Wochen schuppte sich die Haut samt diesen dunkeln, kristallartigen Gebilden ab. Zurück blieben drei helle Muttermale, mit pigmentfreien Stellen, die nicht mehr über das Hautniveau erhaben waren. Fortsetzen der Behandlung führte zu weiterem Verblassen und Abschuppung der Haut an den behandelten Stellen.
c) Behandlung von 2 Naevuszellnaevusvorstufen, das heisst blutunterlaufenen, erhabenen Hautstellen mit 0,3 - 0,4 cm Durchmesser : Eine dreimalige Anwendung des Pflasters mit der 10%-igen Salbe von Bsp. 2e über Nacht führte nach zwei Wochen zu einer Farbveränderung, (hellrotes Gebilde mit dunklen Pünktchen). Die erhabene, veränderte Hautstelle konnte abgelöst werden. Zurück blieben zwei kleine Wunden, infolge zu frühen Ablösens, die verheilten.
d) Eine Probandin mit einem Nävus von ca. 3-4 cm Durchmesser (Basaliom) wendete die 10%-ige Salbe von Bsp. 1 über 3 Monate an (zyklisch, abends, jede 2-te Woche). Der Nävus zeigte anfänglich eine (Entzündungsprozessähnliche) Rötung. Nach 3 Monaten war eine Rückbildung (bezüglich Farbe und Struktur).von ca. 90% erkennbar. Nach ca. 5 Monaten war der Nävus nicht mehr sichtbar.
e) Eine Probandin mit einem Nävus am Arm (Junktionsnaevus) wendete die 10%-ige Salbe von Bsp. 1 an. Da die Probandin nicht überwacht wurde, können hier keine genauen Angaben über die Verabreichungshäufigkeit gemacht werden; die Verabreichung der Salbe estreckte sich aber über mehrere Wochen. Der Nävus ist heute kaum noch erkennbar.
f) Ein 13-jähriger Proband mit einem kongenitalen Nävus im Nasenwurzel-Augenbereich wendete die 10%-ige Salbe von Bsp. 1 2- bis 3-mal wöchentlich unter Okklusion (Pflaster) während zwei Monaten an. Der Nävus wies anfänglich viele kleine dunkle Pünktchen auf. Er reagierte langsamer. Erste Erfolge zeichneten sich allerdings schon ab. Er war insgesamt viel heller geworden und wies mehrere hautfarbene Areale auf.
g) Eine 13-jährige Patientin mit einem dermalen Naevus wendete die 10%-ige Salbe von Bsp.1 während 3 Tagen an. Es erfolgte eine sofortige Konzentration unter Bildung von 3 dunklen Stellen im Nävus, und der restliche Bereich war beinahe farblos.
h) Eine Probandin wendete die 10%-ige Salbe von Bsp. 1 je 1x an 2 aufeinander folgenden Tagen grossflächig im Bauchbereich, wo sie schon zahlreiche Naevuszellnaevi (Junktionsnaevi) hatte, an. Am 2-ten Behandlungstag wurden, zusätzlich zu den bereits bestehenden Nävi, rotel Naevus-Vorstufen der eingangs beschriebenen Art in Form von winzig kleinen Pünktchen sichtbar, die ungleichmässig über die behandelten Flächen verteilt waren. Diese Naevus-Vorstufen waren an sich bereits vorhanden, wurden aber erst durch die Behandlung mit der Salbe sichtbar gemacht. Sie wurden nach 2-3 Tagen dunkel bis schwarz. Teilweise sahen sie wie dunkle, in Poren sitzende Kristalle aus. Sie schuppten sich innerhalb von 2-3 Wochen mit der oberen Stratum Corneum Schicht ab oder lösten sich ab, wenn man mit dem Fingernagel daran etwas kratzte.
i) Eine Probandin behandelte ihre Handrücken, auf denen sie etliche Pigmentmale (Leberflecken) unterschiedlicher Grösse hatte, mit der Salbe von Bsp. 1. Die bestehenden Nävi und Naevus-Vorstufen verblassten zusehends und bildeten sich mit zunehmender Behandlungsdauer (7 bzw. 10 Tage) zurück. Die Farbe der Pigmentmale hellte sich nach dem Absetzen der Behandlung auf. Nach vier Wochen waren sie kaum noch bis nicht mehr sichtbar.

## Patentansprüche

1. Verwendung einer Verbindung der Formel 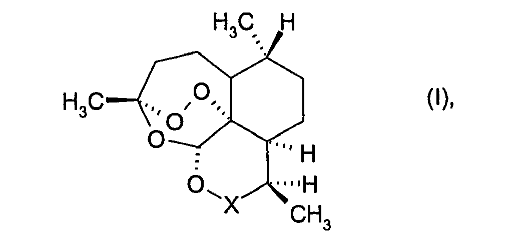 worin
X CO; CHOZ; oder CHNRZ ist,
Z Wasserstoff; ein geradkettiger oder verzweigter (C₁-C₆)Alkyl-, (C₂-C₆)Alkenyl- oder (C₂-C₆)-Alkinyl-Rest; (C₃-C₈)Cycloalkyl; (C₆-C₂₄)Aryl; (C₇-C₂₄)Aralkyl; m- oder p-CH₂(C₆H₄)COOM; COR³; CSR³; C(NR⁶)R³; SOR⁴; SO₂OM; SO₂NR**⁷**R⁸; SO₂O-Artemisinyl; SO₂NH-Artemisinyl; POR⁴R⁵; oder PSR⁴R⁵ ist,
R³ ein geradkettiger oder verzweigter (C₁-C₆)Alkyl-, (C₁-C₆)Alkoxy-, (C₂-C₆)Alkenyl- oder (C₂-C₆)Alkinyl-Rest; (C₃-C₈)Cycloalkyl; (C₆-C₂₄)Aryl; (C₆-C₁₀)Aryloxy; (C₇-C₂₄)Aralkyl; -(CH₂)ₙ-COOM, worin n eine ganze Zahl von 1 bis 6 ist; oder 10α-Dihydroartemisinyl ist,
R⁴ und R⁵, unabhängig voneinander, für einen geradkettigen oder verzweigten (C₁-C₆)Alkyl-, (C₂-C₆)Alkenyl-, (C₁-C₆)Alkoxy- oder (C₂-C₆)Alkinyl-Rest; (C₃-C₈)Cycloalkyl; (C₆-C₂₄)Aryl; (C₇-C₂₄)Aralkyl; OM; (C₆-C₁₀)Aryloxy; oder NR⁷R⁸ stehen,
R⁶ ein geradkettiger oder verzweigter (C₁-C₆)Alkyl-, (C₂-C₆)Alkenyl- oder (C₂-C₆)Alkinyl-Rest; (C₃-C₈)Cycloalkyl; (C₆-C₂₄)Aryl; oder (C₇-C₂₄)Aralkyl ist,
M Wasserstoff; oder ein pharmazeutisch annehmbares Kation ist,
R⁷ und R⁸, unabhängig voneinander, für Wasserstoff; oder geradkettiges oder verzweigtes (C₁-C₆)Alkyl stehen oder, zusammen, (C₄-C₆)Alkylen bilden und
R Wasserstoff; oder R⁶ ist,
zur Herstellung einer lokal applizierbaren, bevorzugt topischen, Formulierung gegen gutartige Pigmentmale oder zur Prävention von Hautkrebs.

2. Verwendung gemäss Anspruch 1, **dadurch gekennzeichnet, dass** die Pigmentmale ausgewählt sind aus erworbenenen Naevuszellnaevi; kongenitalen Naevuszellnaevi; Lentiginosen, insbesondere Leberflecken, Sonnenflecken oder Altersflecken; Störungen der Melaninpigmentierungen, etwa Sommersprossen; und Pigmentmalen der Schleimhäute.

3. Verwendung gemäss Anspruch 2, **dadurch gekennzeichnet, dass** die Pigmentmale erworbene Naevuszellnaevi; kongenitale Naevuszellnaevi; oder Leberflecken sind.

4. Verwendung einer Verbindung wie in Anspruch 1 definiert der Formel I zur Herstellung einer lokal applizierbaren, insbesondere topischen, Formulierung zur Prävention von erworbenen Naevuszellnaevi.

5. Verwendung gemäss einem der Ansprüche 1-4, **dadurch gekennzeichnet, dass** die Verbindung der Formel I Artemisinin; Dihydroartemisinin; ein carboxylgruppenhaltiges Derivat der Formel I, insbesondere Artesunat; Artemether; Arteether; das Propylcarbonat des Dihydroartemisinins; oder Artelinsäure ist.

6. Verwendung gemäss Anspruch 5, **dadurch gekennzeichnet, dass** die Verbindung der Formel I Artemisinin; Dihydroartemisinin; oder Artesunat ist.

7. Verwendung gemäss einem der Ansprüche 1-6, **dadurch gekennzeichnet, dass** die lokal applizierbare, insbesondere topische, Formulierung auf einem Pflaster aufgebracht vorliegt.

8. Pflaster mit einer topischen Formulierung zur Behandlung von gutartigen Pigmentmalen oder zur Prävention von Hautkrebs, **dadurch gekennzeichnet, dass** die Formulierung einen Wirkstoff der Formel 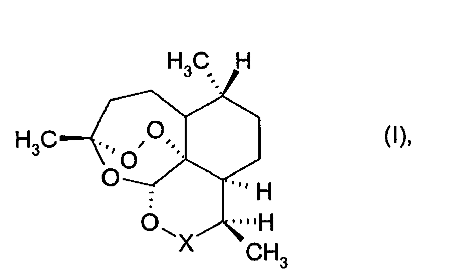 worin X CHOZ; oder CHNRZ ist und Z eine der in Anspruch 1 definierten Bedeutungen hat, enthält und im Wesentlichen frei von penetrationsverstärkenden Stoffen ist.

9. Pflaster gemäss Anspruch 8, **dadurch gekennzeichnet, dass** X in der Formel I CHOZ ist, Z m- oder p-CH₂(C₆H₄)COOM; oder COR³ ist und R³ -(CH₂)ₙ-COOM ist.

10. Pflaster gemäss Anspruch 9, **dadurch gekennzeichnet, dass** der Wirkstoff Artesunat ist.

11. Pflaster gemäss Anspruch 10, **dadurch gekennzeichnet, dass** die Formulierung eine Paste, Salbe, Suspension, Lösung oder Creme oder ein Gel oder Spray, insbesondere eine Salbe, ist.

12. Pflaster gemäss einem der Ansprüche 8-11, **dadurch gekennzeichnet, dass** die Pigmentmale ausgewählt sind aus den in Anspruch 2 definierten Pigmentmalen.

## Claims

1. Use of a compound of the formula 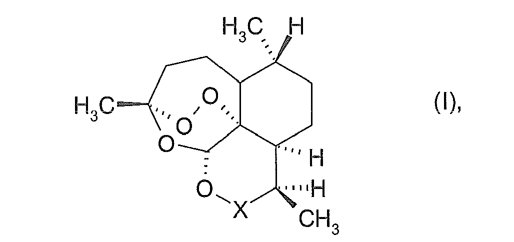 in which
X is CO; CHOZ; or CHNRZ,
Z is hydrogen; a straight-chained or branched (C₁-C₆)alkyl, (C₂-C₆)alkenyl or (C₂-C₆)alkynyl group; (C₃-C₈)cycloalkyl; (C₆-C₂₄)aryl; (C₇-C₂₄)aralkyl; m- or p-CH₂(C₆H₄)COOM; COR³; CSR³; C(NR⁶)R³, SOR⁴; SO₂OM; SO₂NR⁷R⁸; SO₂O-artemisinyl; SO₂NH-artemisinyl; POR⁴R⁵. or PSR⁴R⁵,
R³ is a straight-chained or branched (C₁-C₆)alkyl, (C₁-C₆)alkoxy, (C₂-C₆)alkenyl or (C₂-C₆)-alkynyl group; (C₃-C₈)cycloalkyl; (C₆-C₂₄)aryl; (C₆-C₁₀)aryloxy; (C₇-C₂₄)aralkyl; -(CH₂)ₙ-COOM, in which n is an integer from 1 to 6; or 10α-dihydroartemisinyl,
each of R⁴ and R⁵, independently from one another, is a straight-chained or branched (C₁-C₆)-alkyl, (C₂-C₆)alkenyl, (C₁-C₆)alkoxy or (C₂-C₆)alkynyl group; (C₃-C₈)cycloalkyl; (C₆-C₂₄)aryl; (C₇-C₂₄)aralkyl; OM; (C₆-C₁₀)aryloxy; or NR⁷R⁸,
R⁶ is a straight-chained or branched (C₁-C₆)alkyl, (C₂-C₆)alkenyl or (C₂-C₆)alkynyl group; (C₃-C₈)cycloalkyl; (C₆-C₂₄)aryl; or (C₇-C₂₄)aralkyl,
M is hydrogen; or a pharmaceutically acceptable cation,
each of R⁷ and R⁸, independently from one another, is hydrogen; or straight-chained or branched (C₁-C₆)alkyl, or
R⁷ and R⁸, taken together, are (C₄-C₆)alkylene, and
R is hydrogen; or R⁶,
for the preparation of a locally applicable, preferably topical, formulation against benign pigment marks or for the prevention of skin cancer.

2. Use according to claim 1, **characterized in that** the pigment marks are selected from acquired naevus cell naevi; congenital naevus cell naevi; lentiginoses, in particular liver spots, sun spots or age spots; irregularities of the melanin pigmentations, such as freckles; and pigment marks of the mucous membranes.

3. Use according to claim 2, **characterized in that** the pigment marks are acquired naevus cell naevi; congenital naevus cell naevi; or liver spots.

4. Use of a compound as defined in claim 1 of the formula I for the preparation of a locally applicable, in particular topical, formulation for the prevention of acquired naevus cell naevi.

5. Use according to any one of claims 1 to 4, **characterized in that** the compound of the formula I is artemisinin; dihydroartemisinin; a derivative of the formula I comprising a carboxyl group, in particular artesunate; artemether; arteether; dihydroartemisinin propylcarbonate; or artelinic acid.

6. Use according to claim 5, **characterized in that** the compound of the formula I is artemisinin; dihydroartemisinin; or artesunate.

7. Use according to any one of claims 1 to 6, **characterized in that** the locally applicable, in particular topical, formulation is present on a plaster to which it has been applied.

8. Plaster with a topical formulation for the treatment of benign pigment marks or for the prevention of skin cancer, **characterized in that** the formulation comprises an active ingredient of the formula 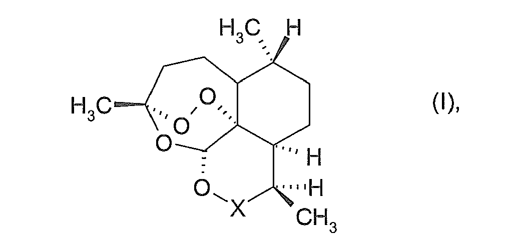 in which X is CHOZ; or CHNRZ, and Z has one of the meanings defined in claim 1, and the formulation is essentially free from penetration-enhancing substances.

9. Plaster according to claim 8, **characterized in that** X in the formula I is CHOZ, Z is m- or p-CH₂(C₆H₄)COOM; or COR³, and R³ is -(CH₂)ₙ-COOM.

10. Plaster according to claim 9, **characterized in that** the active ingredient is artesunate.

11. Plaster according to claim 10, **characterized in that** the formulation is a paste, an ointment, a suspension, a solution, a gel, a spray or a cream, in particular an ointment.

12. Plaster according to any one of claims 8 to 11, **characterized in that** the pigment marks are selected from the pigment marks defined in claim 2.

## Revendications

1. Utilisation d'un composé de la formule 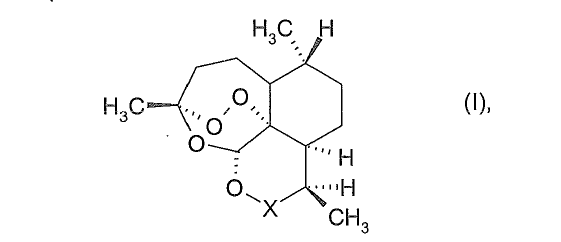 où
X représente CO; CHOZ; ou CHNRZ,
Z représente hydrogène; un radical (C₁-C₆)alkyle, (C₂-C₆)alcényle ou (C₂-C₆)alcynyle, qui est linéaire ou ramifié; (C₃-C₈)cydoalkyle; (C₆-C₂₄)aryle; (C₇-C₂₄)aralkyle; m- ou p-CH₂(C₆H₄)COOM; COR³; CSR³; C(NR⁶)R³; SOR⁴; SO₂OM; SO₂NR⁷R⁸; SO₂O-artémisinyle; SO₂NH-artémisinyle; POR⁴R⁵; ou PSR⁴R⁵,
R³ représente un radical (C₁-C₆)alkyle, (C₁-C₆)alkoxy, (C₂-C₆)alcényle ou (C₂-C₆)alcynyle, qui est linéaire ou ramifié; (C₃-C₈)cycloalkyle; (C₆-C₂₄)aryle; (C₆-C₁₀)aryloxy; (C₇-C₂₄)aralkyle; -(CH₂)ₙ-COOM, où n représente un nombre entier entre 1 et 6; ou 10α-dihydroartémisinyle,
R⁴ et R⁵, indépendamment l'un de l'autre, représentent un radical (C₁-C₆)alkyle, (C₂-C₆)-alcényle, (C₁-C₆)alkoxy ou (C₂-C₆)alcynyle, qui est linéaire ou ramifié; (C₃-C₈)cycloalkyle; (C₆-C₂₄)aryle; (C₇-C₂₄)aralkyle; OM; (C₆-C₁₀)aryloxy; ou NR⁷R⁸
R⁶ représente un radical (C₁-C₆)alkyle, (C₂-C₆)alcényle ou (C₂-C₆)alcynyle, qui est linéaire ou ramifié; (C₃-C₈)cycloalkyle; (C₆-C₂₄)aryle; ou (C₇-C₂₄)aralkyle,
M représente hydrogène; ou un cation pharmaceutiquement acceptable,
R⁷ et R⁸, indépendamment l'un de l'autre, représentent hydrogène; ou (C₁-C₆)alkyle linéaire ou ramifié, ou
R⁷ et R⁸ forment ensemble (C₄-C₆)alcylène, et
R représente hydrogène; ou R⁶
pour la préparation d'une formulation localement applicable, de préférence topique, contre des manifestations pigmentaires bénin ou pour la prévention du cancer de la peau.

2. Utilisation selon la revendication 1, **caractérisée en ce que** les manifestations pigmentaires sont choisies parmi les nævus nævocellulaires acquis; les nævus nævocellulaires congénitaux; les lentiginoses, en particulier les lentigos simplex, lentigos solaris ou lentigos senilis; les troubles de la pigmentation par la mélanine, telles que les éphélides; et les taches pigmentaires des muqueuses.

3. Utilisation selon la revendication 2, **caractérisée en ce que** les manifestations pigmentaires sont les nævus nævocellulaires acquis; les nævus nævocellulaires congénitaux; ou les lentigos simplex.

4. Utilisation d'un composé comme défini dans la revendication 1 de la formule I pour la préparation d'une formulation localement applicable, en particulier topique, pour la prévention de nævus nævocellulaires acquis.

5. Utilisation selon l'une quelconque des revendications 1 à 4, **caractérisée en ce que** le composé de la formule I est de l'artémisinine; du dihydroartémisinine; un dérivé de la formule 1 comprenant un groupe carboxyle, en particulier de l'artésunate; de l'artéméther; de l'artééther; du propyl carbonate du dihydroartémisinine; ou de l'acide artélique.

6. Utilisation selon la revendication 5, **caractérisée en ce que** le composé de la formule I est de l'artémisinine; du dihydroartémisinine; ou de l'artésunate.

7. Utilisation selon l'une quelconque des revendications 1 à 6, **caractérisée en ce que** la formulation localement applicable, en particulier topique, se présente sous une forme appliquée sur un pansement.

8. Pansement avec une formulation topique pour le traitement de manifestations pigmentaires bénin ou pour la prévention du cancer de la peau, **caractérisé en ce que** la formulation comprend une substance active de la formule 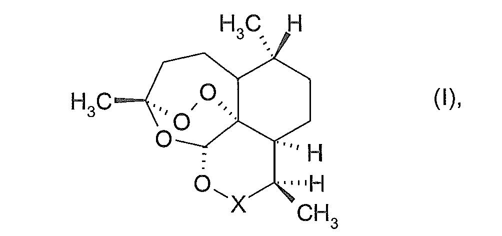 où X représente CHOZ; ou CHNRZ, et Z représente une des significations comme définies dans la revendication 1, et la formulation est essentiellement libre d'agents augmentant le pouvoir de pénétration.

9. Pansement selon la revendication 8, **caractérisé en ce que** X dans la formule I représente CHOZ, Z représente m- ou p-CH₂(C₆H₄)COOM; ou COR³, et R³ représente -(CH₂)ₙ-COOM.

10. Pansement selon la revendication 9, **caractérisé en ce que** la substance active est de l'artésunate.

11. Pansement selon la revendication 10, **caractérisé en ce que** la formulation est sous forme de pâte, pommade, suspension, solution, gel, spray ou crème, en particulier sous forme de pommade.

12. Pansement selon l'une quelconque des revendications 8 à 11, **caractérisé en ce que** les manifestations pigmentaires sont choisies parmi les manifestations pigmentaires comme définies dans la revendication 2.
